(19) Europäisches Patentamt / European Patent Office / Office européen des brevets

(11) **EP 3 176 715 B1**

(12) **FASCICULE DE BREVET EUROPEEN**

(45) Date de publication et mention de la délivrance du brevet:
**12.12.2018 Bulletin 2018/50**

(51) Int Cl.:
***G06F 19/00*** (2018.01)     ***A61B 5/00*** (2006.01)

(21) Numéro de dépôt: **16193041.7**

(22) Date de dépôt: **10.10.2016**

(54) **SYSTÈME DE TRAITEMENT DE DONNÉES POUR LA DÉTECTION D'UNE CRISE D'EXACERBATION CHEZ UN PATIENT SOUFFRANT D'UNE MALADIE RESPIRATOIRE CHRONIQUE TRAITÉ PAR OXYGÉNOTHÉRAPIE**

DATENVERARBEITUNGSSYSTEM ZUR DETEKTION EINER AKUTEN VERSCHLIMMERUNG BEI EINEM PATIENTEN, DER AN EINER CHRONISCHEN ATEMWEGSERKRANKUNG LEIDET, DIE MIT SAUERSTOFFTHERAPIE BEHANDELT WIRD

DATA-PROCESSING SYSTEM FOR DETECTING AN EXACERBATION ATTACK IN A PATIENT SUFFERING FROM A CHRONIC RESPIRATORY DISEASE TREATED BY OXYGEN THERAPY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.12.2015 FR 1561812**

(43) Date de publication de la demande:
**07.06.2017 Bulletin 2017/23**

(73) Titulaire: **L'AIR LIQUIDE, SOCIETE ANONYME POUR L'ETUDE ET L'EXPLOITATION DES PROCEDES GEORGES CLAUDE**
**75007 Paris (FR)**

(72) Inventeurs:
• **AMADOU-BOUBACAR, Habiboulaye**
**78350 JOUY EN JOSAS (FR)**
• **PIGASSOU, Jeanne**
**75008 PARIS (FR)**
• **TEXEREAU, Joëlle**
**75013 PARIS (FR)**

(74) Mandataire: **Pittis, Olivier**
**L'Air Liquide, S.A.**
**Direction de la Propriété Intellectuelle**
**75, Quai d'Orsay**
**75321 Paris Cedex 07 (FR)**

(56) Documents cités:
**WO-A1-2009/094335     WO-A1-2012/131171**
**WO-A1-2015/062811     FR-A1- 2 970 872**
**US-A1- 2013 310 699**

EP 3 176 715 B1

**Description**

La présente invention concerne un système de traitement de données et une installation médicale permettant de suivre à distance, prédire ou détecter au plus tôt, une crise d'exacerbation d'un patient atteint d'une maladie respiratoire chronique, en particulier d'une BPCO, lequel patient est traité par oxygénothérapie, en particulier à son domicile, et d'alerter ensuite le personnel soignant ou analogue de manière à éviter ou à réduire la fréquence de ré-hospitalisation dudit patient.

Les maladies respiratoires chroniques sont en forte progression dans le monde, en particulier la BPCO ou Broncho-Pneumopathie Chronique Obstructive qui affecte les broches des patients, par exemples chez les fumeurs. La BPCO est une maladie chonique dont l'évolution progressive est jalonnée de poussées d'aggravation des symptômes récurrents de la maladie, appelées crises d'exacerbations ou plus simplement exacerbations, notamment une augmentation de la toux et de l'essoufflement.

La répétition des exacerbations aggrave le pronostic de la maladie, donc les risques vitaux du patient. De ce fait, en cas de crise aiguë de BPCO ou analogue, le patient est généralement hospitalisé pour être traité, notamment soigné par administration d'oxygène gazeux, également appelé oxygénothérapie.

Or, on comprend aisément qu'une telle hospitalisation engendre un processus lourd et coûteux, voire même parfois traumatisant pour le patient qui doit passer plusieurs jours en hôpital.

Ensuite, après son hospitalisation, le patient rentre à son domicile où il doit généralement suivre un traitement par oxygénothérapie basé sur une administration d'oxygène gazeux, typiquement produit par un concentrateur d'oxygène ou de tout autre système de fourniture d'oxygène utilisable au domicile du patient.

Pour éviter ou minimiser la fréquence de rechute et donc de ré-hospitalisation des patients, il convient de pouvoir détecter le plus tôt possible la survenance d'une nouvelle crise de BPCO ou analogue chez les patients, c'est-à-dire de pouvoir prédire les exacerbations de manière à permettre une prise en charge des patients avant une crise et éviter leur re-hospitalisation.

Or, ce n'est pas chose aisée, notamment du fait que les patients se trouvent à leur domicile et que l'on doit donc pouvoir détecter les crises d'exacerbations rapidement et surtout à distance. Plusieurs documents se sont déjà attelés à ce problème et ont proposés des solutions qui se sont avérées en pratique rarement efficaces.

Ainsi, le document Chest; Dec. 2012;142(6):1524-9 ; Monitoring breathing rate at home allows early identification of COPD exacerbations; A.M. Yanez et al, propose de prédire les exacerbations de BPCO à partir de l'analyse de la fréquence respiratoire du patient. En effet, il a été montré que la fréquence respiratoire augmente chez 70% des 30 patients testés, durant les 5 jours précédant une exacerbation. Or, la fréquence respiratoire seule n'est pas suffisante pour rendre la prédiction fiable.

Par ailleurs, WO-A-2015062811 propose d'effectuer des mesures de débit et de pression du flux de gaz administré au patient pour en extraire une fréquence respiratoire qui est ensuite analysée. Un algorithme embarqué dans le capteur est chargé de détecter les exacerbations en utilisant la méthode de Young's C Statistic. Là encore, la fréquence respiratoire seule n'est pas suffisante pour rendre la prédiction fiable.

D'autres documents proposent d'exploiter d'autres paramètres.

Ainsi, US-A-2013/030258 propose une méthode de détection de l'exacerbation de BPCO basée sur des critères prédéfinis relatifs à des changements de fréquence respiratoire et de fréquence cardiaque en fonction de variations de l'activité physique du patient.

US-A-2013/0310699 propose une méthode basée sur le recueil de signaux mesurés par des électrodes placées sur les muscles thoraciques du patient, lesquels servent à déterminer différents paramètres de suivi, notamment les rythmes cardiaque et respiratoire, qui permettent de détecter les détériorations de la maladie.

US-A-2014/0221782 propose une méthode théorique utilisant le niveau d-saturation en oxygène dans le sang du patient mesuré par oxymétrie. Un algorithme de prédiction effectue une régression sur les données sur une fenêtre temporelle glissante de 30 jours pour estimer si le patient s'approche d'une exacerbation.

US-A-2014/0206949 décrit un système utilisant un ou plusieurs dispositifs, notamment un spiromètre, un oxymètre pulsé, un thermomètre et un capteur au peroxyde, mettant en oeuvre une méthode de prédiction se basant sur un réseau bayésien pour fournir une probabilité d'exacerbation. Le patient doit effectuer lui-même les mesures.

US-A-2011/0184250 enseigne de réaliser d'abord des mesures des conditions ambiantes dans l'environnement du patient, notamment qualité d'air, niveau d'allergènes, température et conditions atmosphériques. Un algorithme prédit la probabilité d'une exacerbation de BPCO en fonction de l'activité attendue pour le patient dans les conditions ambiantes locales, et alerte le patient des risques avant la période concernée.

Toutefois, ces différentes solutions ne sont pas totalement satisfaisantes puisqu'elles ne permettent pas, elles non plus, de prédire de manière fiable et efficace, les exacerbations BPCO ou analogue chez les patients suivants un traitement d'oxygénothérapie, en particulier des patients traités à domicile.

En outre, aucune de ces solutions ne prend en compte, parmi les variables utilisées, l'observance du patient à son traitement, c'est-à-dire la durée de respiration du patient sous oxygène, qui s'avère être une variable très intéressante, voire essentielle, pour détecter de façon fiable l'avènement d'une crise d'exacerbation.

Certaines de ces solutions ne permettent pas de suivre le patient à distance et/ou nécessitent que le patient réalise lui-même certaines mesures. Ce n'est pas pratique et engendre des risques d'erreur de mesure. D'autres ne sont pas concluantes lorsqu'on les met en oeuvre puisqu'on constate en pratique que beaucoup d'exacerbations ne sont pas détectées par ces méthodes. D'autres encore sont très théoriques et difficilement applicables au domicile d'un patient car trop contraignantes et/ou non-adaptées à un patient actif, c'est-à-dire qui déambule.

Au vu de cela, le problème qui se pose est dès lors de pouvoir détecter au plus tôt, c'est-à-dire prédire, une détérioration de l'état de santé d'un ou plusieurs patients atteints d'une maladie respiratoire, en particulier une BPCO, lesquels patients sont traités par oxygénothérapie à leur domicile, et en réponse à cette détection, d'alerter les professionnels de santé ou analogue pour qu'il puisse prendre des mesures de traitement efficaces, le plus tôt possible, de manière à éviter ou à réduire la fréquence des ré-hospitalisations de ces patients dues aux exacerbations répétées de la BPCO ou analogue.

La solution de l'invention est alors un système de traitement de données permettant de prédire une crise d'exacerbation d'un patient atteint d'une maladie respiratoire chronique, en particulier d'une BPCO, et traité par oxygénothérapie selon les caractéristiques indiquées dans les revendications.

L'invention concerne aussi une installation de suivi de patient permettant de, c'est-à-dire conçue pour et apte à, détecter une crise d'exacerbation d'un patient atteint d'une maladie respiratoire chronique, en particulier d'une BPCO, lequel patient est traité par oxygénothérapie, en particulier à son domicile, caractérisée en ce qu'elle comprend une source de gaz reliée fluidiquement à une interface respiratoire de distribution de gaz au patient de manière à alimenter ladite interface respiratoire de distribution de gaz avec du gaz délivré par la source de gaz, et un boitier d'un système de traitement de données selon l'une des revendications agencé sur le trajet du gaz entre la source de gaz et l'interface respiratoire de distribution de gaz.

La présente invention propose donc un système de traitement de données conçu pour et apte à prédire une crise d'exacerbation grâce à la détection de plusieurs vecteurs hors du modèle mathématique représentatif de l'état de santé normal du patient sur une période de temps définie, typiquement plusieurs jours, idéalement 2 vecteurs hors modèle sur une période maximale de 5 jours consécutifs.

L'invention va maintenant être mieux comprise grâce à la description détaillée suivante, faite à titre illustratif mais non limitatif, en référence aux figures annexées parmi lesquelles :

- la Figure 1 est un schéma d'un mode de réalisation d'une installation médicale de suivi d'au moins un patient atteint d'une maladie respiratoire chronique selon l'invention,

- les Figures 2a et 2b représentent des comparaisons entre la fréquence respiratoire et la durée de respiration, c'est-à-dire l'observance du patient au traitement d'oxygénothérapie, chez un patient en situation normale et dans les jours précédents une crise d'exacerbation de BPCO.

- la Figure 3A est une représentation du modèle mathématique calculé à partir de plusieurs vecteurs tridimensionnels extraits pendant l'état de santé normale d'un patient.

- la Figure 3B est une représentation de l'état du patient jour après jour par des vecteurs tridimensionnels.

- la Figure 4 illustre les cas de détection (cas 3 et 4) ou de non-détection (cas 1 et 2) de détérioration de l'état d'un patient sur une période de 5 jours consécutifs conduisant à prédire ou non une crise d'exacerbation future,

- la Figure 5 illustre un premier mode de réalisation d'un système de traitement de données permettant de prédire une crise d'exacerbation d'un patient atteint d'une maladie respiratoire chronique, en particulier d'une BPCO, et traité par oxygénothérapie selon la présente invention,

- la Figure 6 illustre un second mode de réalisation un seconde mode de réalisation d'un système de traitement de données permettant de prédire une crise d'exacerbation d'un patient atteint d'une maladie respiratoire chronique, en particulier d'une BPCO, et traité par oxygénothérapie selon la présente invention,

- la Figure 7 est un schéma montrant le boitier d'un système de traitement de données selon la présente invention inséré sur le circuit de gaz reliant une source de gaz à une interface patient, et

- la Figure 8 illustre l'extraction du vecteur tridimensionnel en fonction des variables de fréquence respiratoire et durée de traitement.

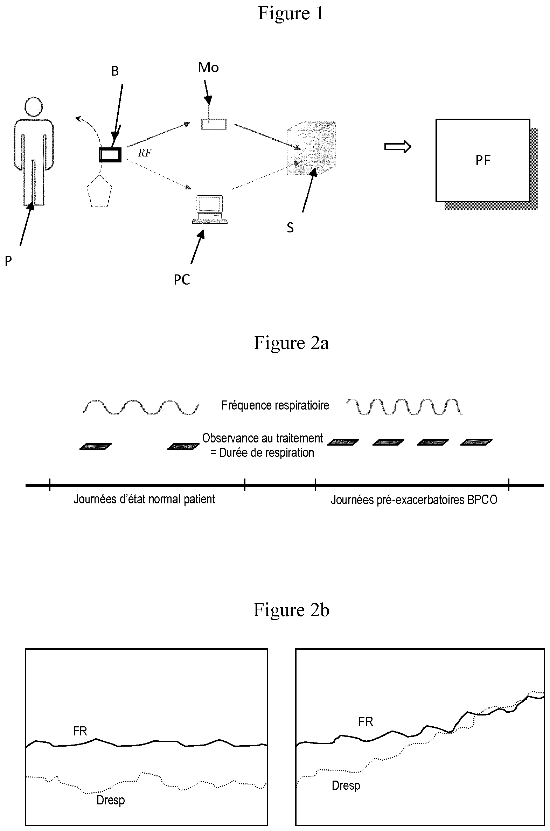

La Figure 1 schématise un mode de réalisation du principe de fonctionnement général d'une installation médicale permettant de suivre à distance, et dès lors de prédire ou détecter au plus tôt, une crise d'exacerbation d'un patient P atteint d'une maladie respiratoire chronique, en particulier d'une BPCO, lequel patient P est traité par oxygénothérapie à son domicile, de manière à éviter ou à réduire la fréquence de ré-hospitalisation de ce patient.

L'installation de suivi comprenant un dispositif d'acquisition et de transmission de données se présentant sous la forme d'un boitier B incluant tout ou partie d'un système de traitement de données selon la présente invention.

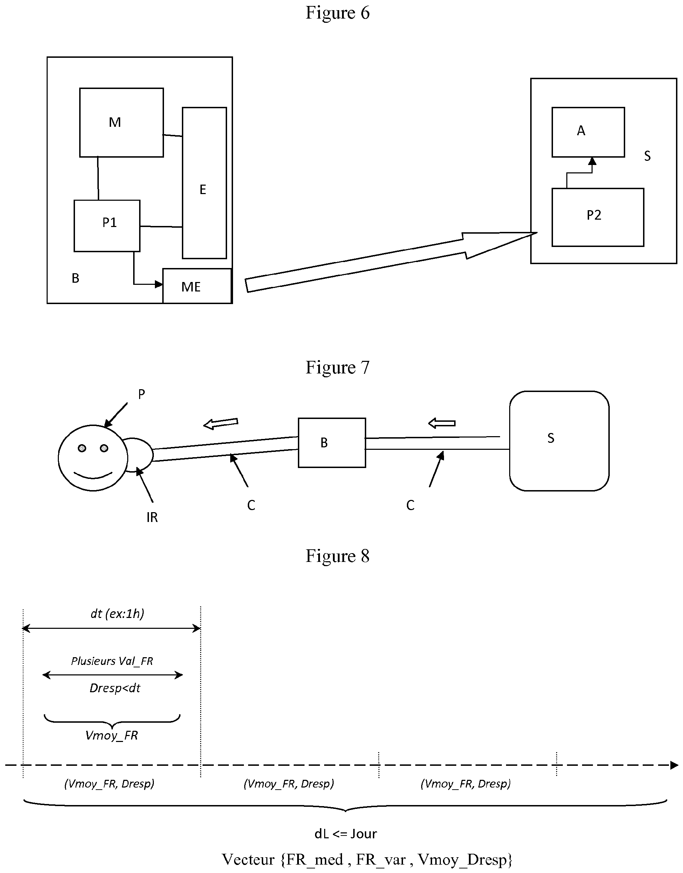

Le boitier B est intercalé, tel que schématisé en Figure 7, sur le trajet du gaz servant au traitement d'oxygénothérapie du patient P, c'est-à-dire typiquement entre une source de gaz d'oxygénothérapie S délivrant typiquement de l'oxygène ou un gaz riche en oxygène (i.e. >80% en vol d'$O_2$), tel un concentrateur d'oxygène, et une interface respiratoire IR fournissant le gaz au patient 30, comme des canules nasales ou un masque respiratoire.

Ainsi, le boitier B est préférentiellement raccordé fluidiquement aux conduits C de gaz, de préférence des tuyaux flexibles ou analogues, servant à véhiculer le gaz depuis la source de gaz S jusqu'à l'interface respiratoire IR.

Plus précisément, le boitier B forme une carcasse externe au sein de laquelle sont agencés un ou plusieurs capteurs de pression de gaz agencé(s) de manière à opérer des mesures de pression au sein d'un conduit de gaz traversant le boitier B et alimenté par la source S de gaz, un ou plusieurs microprocesseurs P1, P2 mettant en oeuvre un (ou plusieurs) algorithme de calcul qui reçoit les signaux de mesure de pression provenant du ou des capteurs de pression et qui les traite pour en déduire des données respiratoires (fréquences, observance ...), des moyens de mémorisation M, telle une mémoire, éventuellement des moyens d'émission à distance des données issues du microprocesseur, et une source d'énergie E électrique, telle une batterie rechargeable ou non.

La source d'énergie E électrique alimente en courant électrique les différents composants nécessitant de l'énergie électrique pour fonctionner, notamment les capteur(s), le(s) microprocesseur(s) P1, P2, les moyens de mémorisation M et éventuellement les moyens d'émission ME de données, par exemple une antenne radiofréquence RF, de manière à permettre à l'ensemble de fonctionner en autonomie. Le rôle des microprocesseurs P1, P2 sera expliqué ultérieurement.

L'architecture interne du boitier B varie selon le mode de réalisation considéré.

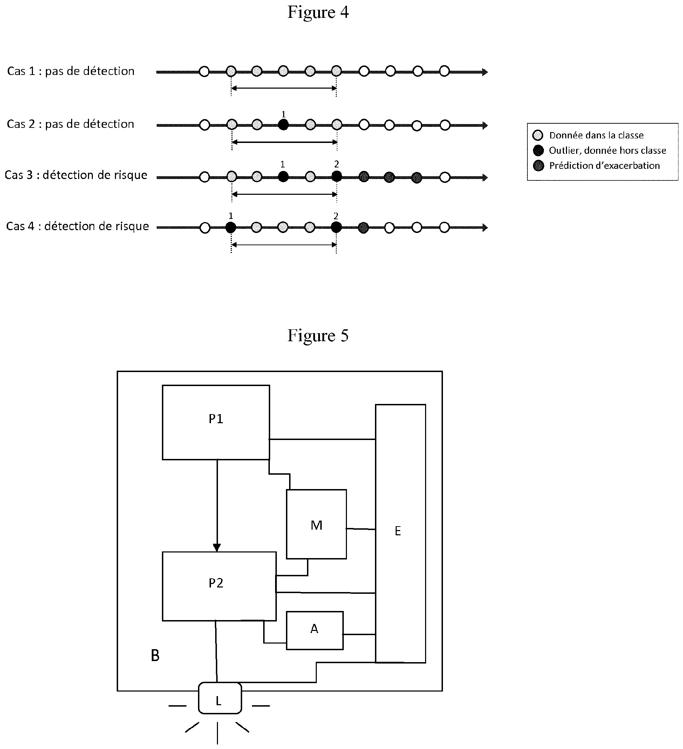

Ainsi, la Figure 5 représente un premier mode de réalisation, dans lequel le boitier B comprend un premier processeur P1 et un deuxième processeur P2 agencés au sein dudit boitier B, en étant reliés par un bus de communication (non montré).

Le deuxième processeur P2 est connecté aux moyens de mémorisation M, telle une puce mémoire (chips) ou analogue. Préférentiellement, le premier processeur P1 peut aussi être connecté aux moyens de mémorisation M. Le premier processeur P1, le deuxième processeur P2 et les moyens de mémorisation M sont alimentés en énergie par la source d'énergie E électrique.

Le boitier B peut aussi comprendre un dispositif lumineux L, de préférence au moins une lampe de type LED, permettant d'alerter l'utilisateur, comme expliqué ci-après. Par ailleurs, le boitier B peut aussi comprendre un afficheur A de données, tel un écran digital. Le dispositif lumineux L et/ou l'afficheur A sont aussi alimentés en électricité par la source d'énergie E électrique.

Par ailleurs, la Figure 6 représente un second mode de réalisation, dans lequel le boitier B comprend, comme précédemment, un premier processeur P1, les moyens de mémorisation M, la source d'énergie E électrique, mais aussi des moyens d'émission ME de données, par exemple une antenne radiofréquence RF, qui sont alimentés en électricité par la source d'énergie E électrique.

Dans ce cas, le deuxième processeur P2 se trouve au sein d'un serveur S distant qui communique à distance avec le boitier B grâce aux moyens d'émission ME de données. Le serveur S peut être connecté à un afficheur A de données, tel un écran d'ordinateur ou autre permettant à une personne de prendre connaissance des informations transmises au serveur S par le boitier B et/ou de données traitées au sein du serveur S, y compris d'alarmes. Au sein du serveur S, les données transmises par le boitier B peuvent être traitées, analysées, comparées etc... de manière à détecter une dégradation de l'état de santé du patient P et prédire une crise imminente d'exacerbation de BPCO.

Dans ce deuxième mode de réalisation, la présente invention permet une surveillance à distance d'un ou de plusieurs patients soignés à leur domicile, grâce à la transmission en temps-réel de données respiratoires par le boitier B, au serveur S distant, tel un serveur ASIP (hébergement agréé pour la santé), comme illustré en Figure 1. La transmission des données vers le serveur distant 1 est opérée via un ordinateur PC ou un modem Mo, par exemple de type GSM.

En d'autres termes, le boitier B opère d'abord des mesures de pression P du flux de gaz d'oxygénothérapie circulant, pendant une durée donnée dt, dans le passage ou conduit traversant le boitier B de manière à pouvoir en déduire, c'est-à-dire déterminer, à l'aide du microprocesseur P1, des variations de pression Dp du flux de gaz pendant la durée donnée dt, à partir de ces mesures de pression P. La durée donnée dt peut être de 15 à 60 minutes.

Les mesures de pression P sont opérées par un (ou plusieurs) capteurs de pression qui transmettent les signaux de mesure au microprocesseur P1 où ils sont traités.

Ces variations de pression DP pendant ladite durée donnée dt permettent au microprocesseur P1 de déterminer ensuite une durée de respiration Dresp du patient, pendant la durée donnée dt, et d'en déduire au moins une valeur de fréquence respiratoire Val_FR pendant la durée donnée dt. Cette opération est répétée plusieurs fois pendant la durée de respiration Dresp de manière à obtenir plusieurs valeurs de fréquences respiratoires Val_FR successives.

Le microprocesseur P1 calcule alors au moins une valeur moyenne de fréquence respiratoire Vmoy_FR

sur la durée de respiration Dresp, à partir de plusieurs valeurs de fréquences respiratoires Val_FR successives mesurées pendant la durée de respiration Dresp.

Ensuite, le premier processeur P1 ou un deuxième processeur P2 calcule au moins une valeur moyenne de durée de respiration Vmoy_Dresp à partir de plusieurs durées de respiration Dresp successives, pendant une durée longue dL de maximum 24 heures.

Le premier processeur P1 ou le deuxième processeur P2 détermine, en outre, à partir de plusieurs valeurs moyennes de fréquence respiratoire Vmoy FR obtenues sur la durée longue dL, une valeur médiane FR_med de fréquence respiratoire et une variance FR_var de fréquence respiratoire.

Ensuite, le premier processeur P1 ou le deuxième processeur P2 peut définir un vecteur tridimensionnel FR_med ; FR_var; Vmoy_Dresp à partir des triplets de valeurs médiane de fréquence respiratoire FR_med, de variance de fréquence respiratoire FR_var et de valeur moyenne de durée de respiration Vmoy_Dresp sur la durée longue dL (dL : maxi. 24 h).

Le deuxième processeur P2 peut alors comparer le vecteur tridimensionnel FR_med ; FR_var ; Vmoy_Dresp ainsi obtenu à un modèle mathématique de référence, mémorisé par des moyens de mémorisation M, qui est représentatif de l'état de santé normal du patient considéré, et détecter tout vecteur tridimensionnel FR_med ; FR_var; Vmoy_Dresp hors du modèle mathématique représentatif de l'état de santé normal du patient considéré.

Les moyens de mémorisation M sont en fait conçus pour mémoriser non seulement les valeurs de moyenne de fréquence respiratoire Vmoy_FR et les durées de respiration Dresp successives fournies par le ou les processeurs P1, P2, mais aussi au moins un modèle mathématique de référence représentatif de l'état de santé normal d'un patient considéré.

Le modèle mathématique de référence mémorisé est défini à partir d'un groupe formé de plusieurs vecteurs tridimensionnels préalablement obtenus à partir de n triplets (avec n>10) de valeur médiane de fréquence respiratoire FR med (n), variance de fréquence respiratoire FR_var(n) et de valeur moyenne de durée de respiration Vmoy_Dresp(n), chez le patient considéré, sur une durée de plusieurs jours durant lesquels le patient n'a pas eu de crise d'exacerbation.

Lorsque, pour un patient donné, plusieurs vecteurs tridimensionnels FR_med ; FR_var; Vmoy_Dresp hors du modèle mathématique sont détectés, sur une période donnée de plusieurs jours, par exemple de 5 jours consécutifs ou moins, le processeur P2 est configuré pour émettre une alerte, telle une alarme visuelle ou sonore, par exemple l'activation d'un dispositif lumineux L, comme représenté en Figure 5, de manière à avertir de la détection d'une dégradation de l'état de santé du patient P susceptible de conduire à une crise imminente d'exacerbation de BPCO.

Des moyens de fourniture d'énergie électrique E, telle une ou des batteries ou analogue, alimentent les différents composants du boitier B, en particulier les moyens de mémorisation M et les processeurs P1, P2.

Le mode de fonctionnement du système selon la présente invention est particulièrement innovant en ce qu'il utilise la durée de respiration Dresp du patient comme donnée respiratoire pertinente, en plus de la fréquence respiratoire Val_FR, pour détecter l'imminence d'une exacerbation de BPCO, i.e. une crise de BPCO.

En effet, des essais réalisés, dans le cadre de la présente invention, sur des patients souffrant de BPCO et traités par oxygénothérapie ont montré qu'une augmentation de l'utilisation des équipements de traitement d'oxygénothérapie par les patients sous oxygénothérapie, donc de la durée de respiration Dresp du patient, est, dans une grande majorité des cas, un signe précurseur de l'avènement d'une exacerbation, comme illustré en Figures 2a et 2b.

Plus précisément, les Figures 2a et 2b représentent des comparaisons entre la fréquence respiratoire et la durée de respiration, c'est-à-dire l'observance du patient au traitement, chez un patient en situation normale et dans les jours précédents une crise d'exacerbation de BPCO.

Ces figurent montrent clairement une augmentation non seulement de la fréquence respiratoire FR mais aussi de la durée de respiration (Dresp) ou observance du patient à son traitement dans les jours précédents une exacerbation BPCO.

En effet, la fréquence respiratoire FR et la durée de respiration Dresp, c'est-à-dire la durée traitement d'oxygénothérapie ou observance, augmentent dans les jours précédents une crise d'exacerbation de BPCO (à droite sur Fig. 2a et 2b) par rapport à une situation normale (à gauche sur Fig. 2a et 2b), car le patient tente de compenser une capacité respiratoire insuffisante en utilisant plus fréquemment son dispositif de traitement par oxygénothérapie.

En d'autres termes, il existe un lien de corrélation entre usage accru d'un équipement de traitement d'oxygénothérapie et crise d'exacerbation de BPCO. Dès lors, exploiter ensemble ces données est particulièrement utile pour prédire efficacement les exacerbations, ce qui se fait dans le cadre de l'invention en opérant :

- une normalisation des variables issues de la fréquence respiratoire à l'aide d'un algorithme temps-réel afin d'ajuster les données à un niveau de base standard pour tous les patients et éliminer les différences d'amplitudes respiratoires en état normal, c'est-à-dire hors crise d'exacerbation.

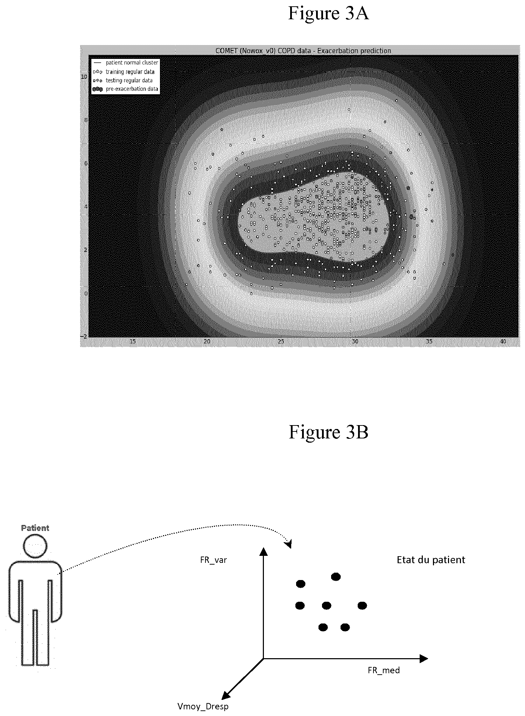

- une définition d'un vecteur multidimensionnel sensible à l'état santé du patient et son risque détérioration, comme illustré en Figure 3A. Il contient les caractéristiques statistiques calculées à partir de la fréquence respiratoire moyenne et l'observance, c'est-à-dire la durée de traitement. Ces caractéristiques sont sélectionnées après plusieurs tests.

- et un apprentissage d'un modèle ou classe caractérisant l'état normal du patient en utilisant les données acquises dans les conditions normales, c'est-à-dire hors exacerbations. Les contours de cette classe sont définis à l'aide d'une fonction mathématique dite « à noyau », comme illustré en Figure 3B.

La méthode est implémentée avec des paramètres réglables et permet donc une meilleure adaptation à la résolution (en anglais : *sparsity*) des données et la prise en compte du bruit dans les données.

Pour un patient donné, lors de l'acquisition de nouvelles données, le risque de dégradation de l'état de santé du patient est identifié en évaluant le signe de la fonction à noyau, c'est-à-dire en comparant les nouvelles données au modèle ou classe caractérisant l'état normal du patient.

Selon l'invention, si la fonction à noyau devient N fois (de préférence N = 2) sur une période de T donnée (de préférence de 5 jours consécutifs), le système de l'invention détecte une détérioration de l'état de santé du patient avec une forte probabilité d'exacerbation dans les prochains jours, comme illustré en Figure 4. Les paramètres N et T sont réglables pour obtenir un bon compromis entre spécificité et sensitivité.

Avantageusement, le système de l'invention implémente un critère de détection plus robuste basé sur une analyse multi-variée comparativement aux méthodes classiques qui sont elles basées sur une analyse de tendance du simple signal de fréquence respiratoire (e.g. la méthode « Young C-statistique »).

Le système de traitement de données selon la présente invention permettant de prédire une crise d'exacerbation d'un patient atteint d'une maladie respiratoire chronique, en particulier d'une BPCO, et traité par oxygénothérapie, présente également les caractéristiques suivantes :

- il permet le suivi de patients à distance jour après jour sans que ces derniers n'interagissent directement avec le dispositif. Le patient n'a besoin ni de porter ou d'installer le dispositif, ni d'enregistrer les données puisque les mesures et le suivi patient se font automatiquement à la mise en route de l'équipement d'oxygénothérapie. Le dispositif est directement intercalé entre le masque et la machine (Fig 1).

- il est configurable pour une détection basée exclusivement sur les données de jour, de nuit, ou des données recueillies sur des journées complètes de 24 h.

- il peut s'interfacer avec une plateforme PF de professionnels de santé (cf. Fig. 1) ou une application mobile pour la gestion d'alertes pour la prévention des événements indésirables comme les exacerbations des patients atteints de BPCO.

Le fonctionnement du système de traitement de données selon la présente invention permettant une détection des détériorations et une prédiction des exacerbations de BPCO repose sur l'acquisition et le prétraitement de données patients ; la représentation de l'état quotidien du patient ; la modélisation de la classe d'état normal du patient; et la détection de risques, comme détaillé ci-après.

### Acquisition et prétraitement des données

Le boitier B mesure la fréquence respiratoire Val_FR à intervalles de temps donnés, par exemple 1 fois toutes les 5 minutes, pour des patients sous oxygénothérapie.

Après chaque durée dl (par exemple 1 heure), sont calculés tout ou partie des paramètres fréquence respiratoire Vmoy_FR sur la durée de respiration Dresp qui est la durée traitement ou d'observance (i.e. détection de respiration). Optionnellement, une phase préalable de prétraitement de données, un algorithme de filtrage est implémenté pour éliminer les données non valides. Il s'agit principalement de valeurs nulles enregistrées hors traitement ou des valeurs correspondantes à des durées très faibles, voire insignifiantes, d'utilisation.

Une fonction de normalisation permet ensuite d'ajuster les données du patient. L'intérêt est d'élaguer les différences d'amplitudes respiratoires dans les conditions normales. La normalisation se fait en temps réel au fur est à mesure de l'acquisition en appliquant l'équation (I) suivante :

$$x' = \frac{x - x_{min}}{x_{max} - x_{min}} \qquad (I)$$

Les variables $x_{min}$ et $x_{max}$ sont estimées sur un échantillon suffisant des données acquises sur le patient dans un état normal, par exemple un échantillon de 1 à 10 jours.

Les données sont ensuite transmises par fréquence radio FR à un ordinateur PC ou à modem GSM Mo relayant vers un serveur S, tel qu'illustré en Figure 1 (transmission dans la Configuration X +YZ). La fréquence de transmission est modulable de l'heure à la journée. Par défaut, les données sont transmises chaque heure.

### Représentation de l'état du patient par un vecteur de caractéristiques

Afin de suivre l'état du patient BPCO, plusieurs variables statistiques sont dérivées des données respiratoires, en particulier la valeur moyenne de la fréquence respiratoire Vmoy_FR et la valeur moyenne de durée de respiration Vmoy_Dresp, et testées pour évaluer leur sensibilité par rapport à l'état de santé du patient.

Les caractéristiques (i.e. paramètres) les plus pertinentes identifiées pour détecter les risques de détérioration sont :

- Valeur médiane de la fréquence respiratoire FR_med ou $x_{FR\_median}$
- Variance de la fréquence respiratoire FR_var ou $x_{FR\_var}$
- Moyenne journalière de la durée de traitement Vmoy_Dresp ou $Tr_{duration}$

En effet, ces trois caractéristiques demeurent relativement stables dans les conditions normales et ont tendance à augmenter les jours précédents une exacerbation.

L'état du patient est donc caractérisé par ces trois variables représentées dans un vecteur multidimensionnel $X^t$ (durée t est réglable), comme illustré sur les Figures 3A, 3B et 8.

Ainsi, la Figure 8 illustre l'extraction du vecteur tridimensionnel en fonction des variables de fréquence respiratoire et durée de traitement. Ce vecteur est ainsi établi pour caractériser quotidiennement l'état de santé du patient.

Le système de l'invention se base dès lors sur le suivi quotidien de ce vecteur multidimensionnel pour permettre une détection d'exacerbation de BPCO.

Prédiction des exacerbations du patient atteint de BPCO

Dans le cadre de l'invention, le modèle prédictif des exacerbations a été développé à partir d'une méthode dite « à noyau » avec une approche d'apprentissage statistique (*Machine Learning*).Voir notamment le document : *Estimating the support of a high-dimensional distribution Schö̈lkopf, Bernhard, et al. Neural computation 13.7 (2001): 1443-1471.*

Cette méthode de prédiction se décompose en une modélisation de la classe état normal d'un patient et une détection des détériorations avec prédiction d'exacerbations en cas de détection d'un état « anormal », comme expliqué ci-après.

i) Modélisation de la classe « état normal »

A partir d'un ensemble de données acquises dans des conditions normales, c'est-à-dire hors de situation de détérioration, est estimé un modèle de classe représentatif de l'état normal du patient. Ce modèle est défini par une fonction dite « à noyau » capable de caractériser des distributions complexes avec une grande robustesse aux bruits présents dans les données.

Cette fonction φ est définie comme suit :

$$\varphi(X^t) = \sum_{i=0}^{n} \alpha_i \cdot \exp\left(-\frac{X^t - X_i}{2\sigma}\right) - \rho$$

où :

- Xi sont les vecteurs de n triplets obtenus en absence de crise,
- $\alpha_i$ sont les coefficients pondérant l'influence des triplets Xi contribuant à la définition du contour de la classe, état normal du patient,
- ρ est un paramètre mathématique représentant la déviation du modèle,
- σ est un paramètre dit du noyau gaussien, et
- Xt est un vecteur tridimensionnel extrait à l'instant et comparer avec le modèle mathématique.

L'estimation de cette fonction est opérée suivant l'approche de minimisation de risque régularisée, telle que décrite par le document V. Vapnik; Statistical Learning Theory ; John Wiley and Sons Inc. ; New York ; 1998.

L'algorithme est implémenté avec un paramètre $\eta$ introduit dans la fonction objective. Il est également appelé facteur de relaxation et contrôle la fraction des données autorisées en déhors du contour.

Les paramètres σ et $\eta$ permettent d'assurer le réglage optimal du modèle en prenant en compte respectivement la distribution (résolution) des données et la robustesse au bruit.

ii) Détection de détériorations et prédiction d'exacerbations

Le système de détection des exacerbations de l'invention se base sur une étude du signe (+) ou (-) de fonction φ :

- $\varphi(X^t) \geq 0$ => donnée dans la classe. Etat normal du patient.
- $\varphi(X^t) < 0$ => donnée hors classe. Risque de détérioration de l'état du patient.

Le critère de détection de détérioration est basé sur la fréquence des cas où $\varphi(X^t) < 0$, c'est-à-dire le nombre n de données détectées hors classe d'état normal sur une fenêtre glissante de plusieurs jours m. Selon l'invention, le meilleur compromis entre spécificité et sensitivité de détection de détérioration est obtenu pour : *n* = 2 sur *m* = 5 jours consécutifs, comme illustré en Figure 4.

Ensuite, la prédiction d'exacerbation se base sur la détection d'une détérioration de l'état du patient. En effet, dans une majorité de cas, cette dégradation se produit chez les patients 5 jours avant l'avenement d'une exacerbation. La détection de détérioration ou prédiction d'exacerbation se produit dans les 1 à 4 jours suivants après que la première donnée est détectée hors de la classe d'état normale, comme illustré en Figure 4.

Ainsi, si aucune détection ou une seule détection de détérioration (« 1 » pour Cas 1 et Cas 2 de la Fig. 4) a lieu pendant 5 jours consécutifs, alors le risque d'exacerbation est considéré comme insuffisant et aucun signalement n'est opéré, c'est-à-dire qu'aucun alarme n'est activée.

A l'inverse, lorsque plusieurs détections de détérioration, c'est-à-dire au moins 2 détections, (« 2 » et « 3 » pour Cas 3 et Cas 4 de la Fig. 4) ont lieu pendant une période maximale de 5 jours consécutifs, c'est-à-dire 5 jours ou moins, alors le risque d'exacerbation chez le patient est élevé et une alerte est émise.

Le système de détection des exacerbations selon l'invention comprend notamment sur un dispositif intégrant un ou plusieurs processeurs, des algorithmes intégrés et des moyens de gestion des alertes.

D'une manière générale, le système de traitement de données de l'invention exploite donc les données non seulement de fréquence respiratoire mais aussi d'observance du patient à son traitement, c'est-à-dire la durée de respiration du patient sous oxygène, pour détecter de façon fiable l'avènement d'une crise d'exacerbation, en particulier en utilisant les valeurs moyennes de fréquence respiratoire Vmoy_FR et de durée de respiration Vmoy_Dresp.

Le système de traitement de données et l'installation médicale selon la présente invention permettent de suivre à distance, prédire ou détecter au plus tôt, une crise d'exacerbation d'un patient atteint d'une maladie respiratoire chronique, en particulier d'une BPCO, lequel patient est traité par oxygénothérapie, en particulier à son domicile, et d'alerter ensuite le personnel soignant ou analogue de manière à éviter ou à réduire la fréquence de ré-hospitalisation de ce patient.

## Revendications

1. Système de traitement de données permettant de prédire une crise d'exacerbation d'un patient atteint d'une maladie respiratoire chronique, en particulier d'une BPCO, et traité par oxygénothérapie, comprenant :

A) au moins un processeur (P1, P2) configuré pour :

a) déterminer des variations de pression (DP) de flux de gaz pendant une durée donnée (dt), à partir de mesures de pression (P) d'un flux de gaz d'oxygénothérapie administré à un patient pendant la durée donnée (dt),
b) déterminer une durée de respiration (Dresp) du patient, avec Dresp < dt, pendant la durée donnée dt, à partir des variations de pression (DP) pendant ladite durée donnée (dt) et en déduire au moins une valeur de fréquence respiratoire (Val_FR) pendant la durée donnée (dt) et répéter plusieurs fois cette détermination de valeur de fréquence respiratoire pendant la durée de respiration (Dresp) de manière à obtenir plusieurs valeurs de fréquences respiratoires (Val_FR) successives mesurées pendant la durée de respiration (Dresp),
c) calculer au moins une valeur moyenne de fréquence respiratoire (Vmoy_FR) sur la durée de respiration (Dresp), à partir de plusieurs valeurs de fréquences respiratoires (Val_FR) successives mesurées pendant la durée de respiration (Dresp),
d) calculer au moins une valeur moyenne de durée de respiration (Vmoy_Dresp) à partir de plusieurs durées de respiration (Dresp) successives pendant une durée longue dL,
e) déterminer, à partir de plusieurs valeurs moyennes de fréquence respiratoire (Vmoy_FR) obtenues sur la durée longue (dL):

- une valeur médiane (FR_med) de fréquence respiratoire et
- une variance (FR_var) de fréquence respiratoire,

f) définir un vecteur tridimensionnel (FR_med ; FR_var; Vmoy_Dresp) à partir des triplets de valeurs médiane de fréquence respiratoire (FR_med), de variance de fréquence respiratoire (FR_var) et de valeur moyenne de durée de respiration (Vmoy_Dresp) sur la durée longue (dL),
g) comparer ou évaluer le vecteur tridimensionnel (FR_med ; FR_var; Vmoy_Dresp) à un modèle mathématique de référence mémorisé représentatif de l'état de santé normal du patient considéré,
h) détecter tout vecteur tridimensionnel (FR_med ; FR_var ; Vmoy_Dresp) hors du modèle mathématique représentatif de l'état de santé normal du patient considéré,

B) des moyens de mémorisation (M) conçus pour mémoriser :

- les valeurs de moyenne de fréquence respiratoire (Vmoy_FR) et les durées de respiration (Dresp) successives fournies par ledit au moins au moins un processeur (P1, P2),
- au moins un modèle mathématique de référence représentatif de l'état de santé normal du patient considéré,

C) et des moyens de fourniture d'énergie électrique (E) alimentant les moyens de mémorisation (M) et ledit au moins un processeur (P1, P2).

**2.** Système selon la revendication précédente, **caractérisé en ce que** ledit au moins au moins un processeur (P1, P2) est configuré pour émettre une alerte lorsque, pour un patient donné, plusieurs vecteurs tridimensionnels (FR med ; FR_var ; Vmoy_Dresp) hors du modèle mathématique sont détectés sur une période donnée de plusieurs jours.

**3.** Système selon la revendication 2, **caractérisé en ce que** la période donnée est d'au moins 2 jours et/ou d'au plus 10 jours, de préférence entre 2 et 8 jours, typiquement de 5 jours.

**4.** Système selon l'une des revendications 2 ou 3, **caractérisé en ce que** le processeur est configuré pour émettre une alerte lorsqu'au moins 2 vecteurs tridimensionnels (FR_med ; FR_var; Vmoy_Dresp) hors du modèle mathématique sont détectés sur une période donnée allant de 2 à 5 jours.

**5.** Système selon la revendication 1, **caractérisé en ce que** le modèle mathématique de référence mémorisé par les moyens de mémorisation (M) est défini à partir d'un groupe formé de plusieurs vecteurs tridimensionnels préalablement obtenus à partir de n triplets (de préférence avec n>10) e valeur médiane de fréquence respiratoire (FR med (n)), variance de fréquence respiratoire (FR_var(n)) et de valeur moyenne de durée de respiration (Vmoy_Dresp(n)), chez le patient considéré, sur une durée de plusieurs jours durant lesquels le patient n'a pas eu de crise d'exacerbation.

**6.** Système selon l'une des revendications précédentes, **caractérisé en ce que** la durée longue (dL) est inférieure ou égale à 24 heures.

**7.** Système selon l'une des revendications précédentes, **caractérisé en ce qu'**il comprend un premier processeur (P1) et un deuxième processeur (P2).

**8.** Système selon l'une des revendications précédentes, **caractérisé en ce qu'**il comprend :

- un premier processeur (P1) configuré pour opérer les opérations a) à c) et un deuxième processeur (P2) configuré pour opérer les opérations d) à h), ou
- un premier processeur (P1) configuré pour opérer les opérations a) à f) et un deuxième processeur (P2) configuré pour opérer les opérations g) à h).

**9.** Système selon l'une des revendications précédentes, **caractérisé en ce qu'**il comprend un premier processeur (P1) et un deuxième processeur (P2) agencés au sein d'un même boitier (B), et un bus de communication agencé entre les premier (P1) et deuxième (P2) processeurs.

**10.** Système selon la revendication 9, **caractérisé en ce que** le boitier (B) comprend un dispositif lumineux (L), de préférence au moins une lampe de type LED.

**11.** Système selon l'une des revendications 1 à 8, **caractérisé en ce qu'**il comprend :

- un premier processeur (P1) agencés au sein d'un boitier (B), ledit boitier (B) comprenant des moyens d'émission (ME) conçus pour transmettre des données, lesdites données comprenant au moins la moyenne de fréquence respiratoire (Vmoy_FR) et la durée de respiration (Vmoy_Dresp), et
- un deuxième processeur (P2) agencé dans un serveur (S) distant conçu pour traiter les données fournies par les moyens d'émission (ME) du boitier (B).

**12.** Système selon l'une des revendications précédentes, **caractérisé en ce qu'**il comprend un dispositif d'affichage de données (A) configuré pour afficher au moins une alerte émise par ledit au moins un processeur (P1, P2) en cas de détection par ledit au moins un processeur (P1, P2) de plusieurs vecteurs tridimensionnels (FR_med ; FR_var ; Vmoy_Dresp) hors du modèle mathématique, de préférence le dispositif d'affichage de données (A) comprend un écran, tel un écran d'ordinateur, de tablette ou de téléphone.

**13.** Système selon l'une des revendications précédentes, **caractérisé en ce que** le boitier (B) comprenant un passage de gaz et un capteur de pression agencé sur ledit passage de gaz de manière à opérer des mesures de pression de gaz au sein dudit passage de gaz.

**14.** Installation de suivi de patient permettant de détecter une crise d'exacerbation d'un patient atteint d'une maladie respiratoire chronique, en particulier d'une BPCO, lequel patient est traité par oxygénothérapie, en particulier à son domicile, **caractérisée en ce qu'**elle comprend une source de gaz (S) reliée fluidiquement à une interface respiratoire (IR) de distribution de gaz au patient de manière à alimenter ladite interface respiratoire (IR) de distribution de gaz avec du gaz délivré par la source de gaz (S), et un boitier (B) d'un système de traitement de données selon l'une des revendications précédentes agencé sur le trajet (C) du gaz entre la source de gaz (IR) et l'interface respiratoire (IR) de distribution de gaz.

**15.** Installation de suivi selon la revendication 16, **caractérisée en ce que** le boitier (B) comprend des moyens d'émission (ME) conçus pour transmettre des données via un dispositif de transmission intermédiaire configuré pour relayer les données vers un serveur distant, de préférence le dispositif de transmission intermédiaire comprend un ordinateur ou un modem, en particulier un modem GSM.

16. Installation de suivi selon l'une des revendications 16 ou 17, **caractérisée en ce que** le boitier (B) comprend des moyens d'émission (ME) conçus pour transmettre des données par radiofréquence ou par Bluetooth™.

## Patentansprüche

1. Datenverarbeitungssystem zur Vorhersage einer akuten Verschlimmerung bei einem Patienten, der an einer chronischen Atemwegserkrankung, insbesondere COPD, leidet, die mit Sauerstofftherapie behandelt wird, umfassend:

   A) mindestens einen Prozessor (P1, P2) konfiguriert zum:

   a) Bestimmen von Druckschwankungen (DP) des Gasstroms während einer gegebenen Dauer (dt), aus Druckmessungen (P) eines Gasstroms zur Sauerstofftherapie, der einem Patienten während der gegebenen Dauer (dt) verabreicht wird,
   b) Bestimmen einer Atemdauer (Dresp) beim Patienten, mit Dresp < dt, während der gegebenen Dauer dt aus den Druckschwankungen (DP) während der gegebenen Dauer (dt) und Ableiten mindestens eines Atemfrequenzwertes (Val_FR) daraus während der gegebenen Dauer (dt) und mehrfaches Wiederholen dieser Bestimmung des Atemfrequenzwertes während der Atemdauer (Dresp), um mehrere aufeinanderfolgende Atemfrequenzwerte (Val_FR), gemessen während der Atemdauer (Dresp), zu erhalten,
   c) Berechnen mindestens eines Mittelwerts der Atemfrequenz (Vmoy_FR) über die Atemdauer (Dresp), aus mehreren aufeinanderfolgenden Atemfrequenzwerten (Val_FR), die während der Atemdauer (Dresp) gemessen wurden,
   d) Berechnen mindestens eines Mittelwerts der Atemdauer (Vmoy_Dresp) aus mehreren aufeinanderfolgenden Atemzeiten (Dresp) über einen langen Zeitraum dL,
   e) Bestimmen aus mehreren Mittelwerten der Atemfrequenz (Vmoy_FR) im Langzeitverlauf (dL):

   - eines Medianwerts (FR_med) der Atemfrequenz und
   - einer Varianz (FR_var) der Atemfrequenz,

   f) Definieren eines dreidimensionalen Vektors (FR_med; FR_var; Vmoy_Dresp) aus den Triolen von Mittelwerten der Atemfrequenz (FR_med), Varianz der Atemfrequenz (FR_var) und Mittelwert der Atemdauer (Vmoy_Dresp) über einen langen Zeitraum (dL),
   g) Vergleichen oder Auswerten des dreidimensionalen Vektors (FR_med; FR_var; Vmoy_Dresp) mit einem gespeicherten mathematischen Referenzmodell, das den normalen Gesundheitszustand des betreffenden Patienten darstellt,
   h) Detektieren jedes dreidimensionalen Vektors (FR_med; FR_var; Vmoy_Dresp) außerhalb des mathematischen Modells, das den normalen Gesundheitszustand des betreffenden Patienten darstellt,

   B) Speichermittel (M), die zum Speichern bestimmt sind von:

   - den Mittelwerten der Atemfrequenz (Vmoy_FR) und den aufeinanderfolgenden Atemzeiten (Dresp), die von dem mindestens einen Prozessor (P1, P2) bereitgestellt werden,
   - mindestens einem mathematischen Referenzmodell, das den normalen Gesundheitszustand des betreffenden Patienten darstellt,

   C) und Stromversorgungsmittel (E), welche die Speichermittel (M) und den mindestens einen Prozessor (P1, P2) versorgen.

2. System nach dem vorstehenden Anspruch, **dadurch gekennzeichnet, dass** der mindestens eine Prozessor (P1, P2) so konfiguriert ist, dass er einen Alarm ausgibt, wenn für einen gegebenen Patienten mehrere dreidimensionale Vektoren (FR_med; FR_var; Vmoy_Dresp) außerhalb des mathematischen Modells über einen gegebenen Zeitraum von mehreren Tagen detektiert werden.

3. System nach Anspruch 2, **dadurch gekennzeichnet, dass** der angegebene Zeitraum mindestens 2 Tage und/oder höchstens 10 Tage, vorzugsweise zwischen 2 und 8 Tagen, typischerweise 5 Tage beträgt.

4. System nach einem der Ansprüche 2 oder 3, **dadurch gekennzeichnet, dass** der Prozessor so konfiguriert ist, dass er einen Alarm ausgibt, wenn mindestens zwei dreidimensionale Vektoren (FR_mcd; FR_var; Vmoy_Dresp) außerhalb des mathematischen Modells über einen gegebenen Zeitraum von 2 bis 5 Tagen detektiert werden.

5. System nach Anspruch 1, **dadurch gekennzeichnet, dass** das von den Speichermitteln (M) gespeicherte mathematische Referenzmodell aus einer Gruppe definiert ist, die aus mehreren dreidimensionalen Vektoren gebildet ist, die zuvor aus n Triolen (vorzugsweise mit n>10) und dem Medianwert der Atemfrequenz (FR_med(n)), der Varianz der Atemfrequenz (FR_var(n)) und dem Mittelwert der Atemdauer (Vmoy_Dresp(n)) bei dem betroffenen Patienten über einen Zeitraum von mehreren Tagen erhalten wurden, in dem der Patient keine akute Verschlimmerung hatte.

6. System nach einem der vorstehenden Ansprüche, **dadurch gekennzeichnet, dass** der lange Zeitraum (dL) weniger als oder gleich 24 Stunden beträgt.

7. System nach einem der vorstehenden Ansprüche, **dadurch gekennzeichnet, dass** es einen ersten Prozessor (P1) und einen zweiten Prozessor (P2) umfasst.

8. System nach einem der vorstehenden Ansprüche, **dadurch gekennzeichnet, dass** es Folgendes umfasst:

   - einen ersten Prozessor (P1), der zum Betrieb der Vorgänge a) bis c) konfiguriert ist und einen zweiten Prozessor (P2), der zum Betrieb der Vorgänge d) bis h) konfiguriert ist, oder
   - einen ersten Prozessor (P1), der zum Betrieb der Vorgänge a) bis f) konfiguriert ist und einen zweiten Prozessor (P2), der zum Betrieb der Vorgänge g) bis h) konfiguriert ist.

9. System nach einem der vorstehenden Ansprüche, **dadurch gekennzeichnet, dass** es einen ersten Prozessor (P1) und einen zweiten Prozessor (P2) umfasst, die innerhalb desselben Gehäuses (B) angeordnet sind, und einen Kommunikationsbus, der zwischen dem ersten (P1) und dem zweiten (P2) Prozessor angeordnet ist.

10. System nach Anspruch 9, **dadurch gekennzeichnet, dass** das Gehäuse (B) eine Beleuchtungsvorrichtung (L), vorzugsweise mindestens eine LED-Lampe, umfasst.

11. System nach einem der Ansprüche 1 bis 8, **dadurch gekennzeichnet, dass** es Folgendes umfasst:

    - einen ersten Prozessor (P1), der in einem Gehäuse (B) angeordnet ist, wobei das Gehäuse (B) Übertragungsmittel (ME) umfasst, die geeignet sind, Daten zu übertragen, wobei die Daten mindestens den Mittelwert der Atemfrequenz (Vmoy_FR) und die Atmungsdauer (Vmoy_Dresp) umfassen, und
- einen zweiten Prozessor (P2), der in einem entfernten Server (S) angeordnet ist, der geeignet ist, die von den Übertragungsmitteln (ME) des Gehäuses (B) gelieferten Daten zu verarbeiten.

12. System nach einem der vorstehenden Ansprüche, **dadurch gekennzeichnet, dass** es eine Datenanzeigevorrichtung (A) umfasst, die so konfiguriert ist, dass sie mindestens einen Alarm anzeigt, der von dem mindestens einen Prozessor (P1, P2) bei Detektion mehrerer dreidimensionaler Vektoren (FR_med; FR_var; Vmoy_Dresp) durch den mindestens einen Prozessor (P1, P2) außerhalb des mathematischen Modells ausgegeben wird, wobei die Datenanzeigevorrichtung (A) vorzugsweise einen Bildschirm, wie einen Computer-, Tablet- oder Telefonbildschirm umfasst.

13. System nach einem der vorstehenden Ansprüche, **dadurch gekennzeichnet, dass** das Gehäuse (B) einen Gasdurchgang und einen Drucksensor umfasst, der an dem Gasdurchgang angeordnet ist, um Gasdruckmessungen innerhalb des Gasdurchgangs durchzuführen.

14. Patientenüberwachungseinrichtung zur Detektion einer akuten Verschlimmerung bei einem Patienten, der an einer chronischen Atemwegserkrankung, insbesondere COPD, leidet, die mit Sauerstofftherapie, insbesondere bei sich zu Hause, behandelt wird, **dadurch gekennzeichnet, dass** es eine Gasquelle (S), die mit einer Beatmungsschnittstelle (IR) zur Gasverteilung an den Patienten fluidisch verbunden ist, um die Beatmungsschnittstelle (IR) zur Gasverteilung mit Gas zu versorgen, das von der Gasquelle (S) geliefert wird, und ein Gehäuse (B) eines Datenverarbeitungssystems nach einem der vorstehenden Ansprüche umfasst, das auf dem Gasweg (C) zwischen der Gasquelle (IR) und der Atmungsschnittstelle (IR) zur Gasverteilung angeordnet ist.

15. Überwachungseinrichtung nach Anspruch 16, **dadurch gekennzeichnet, dass** das Gehäuse (B) Übertragungsmittel (ME) umfasst, die geeignet sind, Daten über eine Zwischenübertragungsvorrichtung zu übertragen, die dazu konfiguriert ist, die Daten an einen entfernten Server weiterzuleiten, vorzugsweise umfasst die Zwischenübertragungsvorrichtung einen Computer oder ein Modem, insbesondere ein GSM-Modem.

16. Überwachungseinrichtung nach einem der Ansprüche 16 oder 17, **dadurch gekennzeichnet, dass** das Gehäuse (B) Übertragungsmittel (ME) umfasst, die geeignet sind, Daten per Funkfrequenz oder per Bluetooth™ zu übertragen.

**Claims**

1. Data processing system enabling to predict an exacerbation crisis for a patient suffering from chronic respiratory disease, in particular a COPD, and treated by oxygen therapy, comprising:

    A) at least one processor (P1, P2) configured to:

        a) determine gas flow pressure variations (DP) for a given duration (dt), from pressure measurements (P) of a gas flow from oxygen therapy administered to a patient for the given duration (dt),
        b) determine a respiration duration (Dresp) of the patient, with Dresp < dt, for the given duration dt, from the pressure variations (DP) for said given duration (dt) and by deducing at least one respiratory frequency value (Val_FR) for the given duration (dt) and repeating several times this determination of respiratory frequency value for the respiration duration (Dresp) so as to obtain several successive respiratory frequency values (Val_FR) measured during the respiration duration (Dresp),
        c) calculate at least one average respiratory frequency value (Vmoy_FR) over the respiration duration (Dresp), from several successive respiratory frequency values (Val_FR) measured during the respiration duration (Dresp),
        d) calculate at least one average respiratory duration value (Vmoy_Dresp) from several successive respiration durations (Dresp) for a long duration dL,
        e) determine, from several average respiratory frequency values (Vmoy_FR) obtained over the long duration (dL):

            - a median respiratory frequency value (FR_med) and
            - a respiratory frequency variance (FR_var),

        f) define a three-dimensional vector (FR_med; FR_var; Vmoy_Dresp) from the triplets of median respiratory frequency values (FR_med), respiratory frequency variance (FR_var) and the average respiration duration value (Vmoy_Dresp) over the long duration (dL),

        g) compare or evaluate the three-dimensional vector (FR_med; FR_var; Vmoy_Dresp) from a memorised mathematical reference model representative of the state of normal health of the patient considered,
        h) detect any three-dimensional vector (FR_med; FR_var; Vmoy_Dresp) outside of the mathematical model representative of the state of normal health of the patient considered,

    B) memorisation means (M) designed to memorise:

        - the average respiratory frequency values (Vmoy_FR) and the successive respiration durations (Dresp) provided by said at least one processor (P1, P2),
        - at least one mathematical reference model representative of the state of normal health of the patient considered,

    C) and means for supplying electrical energy (E), supplying the memorisation means (M) and said at least one processor (P1, P2).

2. System according to the preceding claim, **characterised in that** said at least one processor (P1, P2) is configured to emit an alert when, for a given patient, several three-dimensional vectors (FR_med; FR_var; Vmoy_Dresp) outside of the mathematical model are detected over a given period of several days.

3. System according to claim 2, **characterised in that** the given period is at least 2 days and/or at the most 10 days, preferably between 2 and 8 days, typically 5 days.

4. System according to one of claims 2 or 3, **characterised in that** the processor is configured to emit an alert when at least 2 three-dimensional vectors (FR_med; FR_var; Vmoy_Dresp) outside of the mathematical model are detected over a given period going from 2 to 5 days.

5. System according to claim 1, **characterised in that** the mathematical reference model memorised by the memorisation means (M) is defined from a group formed of several three-dimensional vectors previously obtained from n triplets (preferably with n>10)e median respiratory frequency value (FR_med(n)), respiratory frequency variance (FR_var(n)) and average respiration duration value (Vmoy_Dresp(n)), in the patient considered, over a duration of several days during which the patient has had no exacerbation crisis.

**6.** System according to any one of the preceding claims, **characterised in that** the long duration (dL) is less than or equal to 24 hours.

**7.** System according to any one of the preceding claims, **characterised in that** it comprises a first processor (P1) and a second processor (P2).

**8.** System according to any one of the preceding claims, **characterised in that** it comprises:

- a first processor (P1) configured to carry out the operations a) to c) and a second processor (P2) configured to carry out the operations d) to h), or
- a first processor (P1) configured to carry out the operations a) to f) and a second processor (P2) configured to carry out the operations g) to h).

**9.** System according to any one of the preceding claims, **characterised in that** it comprises a first processor (P1) and a second processor (P2) arranged within one same casing (B), and a communication bus arranged between the first (P1) and second (P2) processors.

**10.** System according to claim 9, **characterised in that** the casing (B) comprises a light device (L), preferably at least one LED-type lamp.

**11.** System according to any of claims 1 to 8, **characterised in that** it comprises:

- a first processor (P1) arranged within one casing (B), said casing (B) comprising emission means (ME) designed to transmit data, said data comprising at least the average respiratory frequency (Vmoy_FR) and the respiration duration (Vmoy_Dresp), and
- a second processor (P2) arranged in a remote server (S) designed to process the data provided by the emission means (ME) of the casing (B).

**12.** System according to any one of the preceding claims, **characterised in that** it comprises a device for displaying data (A) configured to display at least one alert emitted by said at least one processor (P1, P2) in case of detection by said at least one processor (P1, P2) of several three-dimensional vectors (FR_med; FR_var; Vmoy_Dresp) outside of the mathematical model, preferably the device for displaying data (A) comprises a screen, such as a computer, tablet or telephone screen.

**13.** System according to any one of the preceding claims, **characterised in that** the casing (B) comprising a gas passage and a pressure sensor arranged over said gas passage so as to carry out gas pressure measurements within said gas passage.

**14.** Patient monitoring installation enabling to detect an exacerbation crisis of a patient suffering from a chronic respiratory disease, in particular a COPD, which patient is treated by oxygen therapy, in particular at their home, **characterised in that** it comprises a gas source (S) connected fluidly to a respiratory interface (IR) for distributing gas to the patient so as to supply said gas distribution respiratory interface (IR) with gas delivered by the gas source (S), and a casing (B) for a data processing system according to any one of the preceding claims arranged over the gas path (C) between the gas source (IR) and the gas distribution respiratory interface (IR).

**15.** Monitoring installation according to claim 16, **characterised in that** the casing (B) comprises emission means (ME) designed to transmit data via an intermediate transmission device configured to relay data to a remote server, preferably the intermediate transmission device comprises a computer or a modem, in particular a GSM modem.

**16.** Monitoring installation according to any of the claims 16 or 17, **characterised in that** the casing (B) comprises emission means (ME) designed to transmit data by radiofrequency or by Bluetooth™.

## Figure 1

## Figure 2a

Fréquence respiratioire

Observance au traitement
= Durée de respiration

Journées d'état normal patient

Journées pré-exacerbatoires BPCO

## Figure 2b

FR

Dresp

FR

Dresp

Figure 3A

Figure 3B

Figure 4

Cas 1 : pas de détection

Cas 2 : pas de détection

Cas 3 : détection de risque

Cas 4 : détection de risque

○ Donnée dans la classe
● Outlier, donnée hors classe
● Prédiction d'exacerbation

Figure 5

Figure 6

M

E

P1

B

ME

A

S

P2

Figure 7

P

IR

C

B

C

S

Figure 8

*dt (ex:1h)*

*Plusieurs Val_FR*

*Dresp<dt*

*Vmoy_FR*

*(Vmoy_FR, Dresp)*          *(Vmoy_FR, Dresp)*          *(Vmoy_FR, Dresp)*

dL <= Jour

Vecteur {FR_med , FR_var , Vmoy_Dresp}

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015062811 A
- US 2013030258 A
- US 20130310699 A
- US 20140221782 A
- US 20140206949 A
- US 20110184250 A

**Littérature non-brevet citée dans la description**

- **A.M. YANEZ.** Monitoring breathing rate at home allows early identification of COPD exacerbations. *Chest,* Décembre 2012, vol. 142 (6), 1524-9
- **LKOPF ; BERNHARD et al.** *Neural computation,* 2001, vol. 13.7, 1443-1471
- **V. VAPNIK.** Statistical Learning Theory. John Wiley and Sons Inc, 1998